# FASCICULE DE BREVET EUROPEEN

(11) **EP 3 024 414 B1**
(45) Date de publication et mention de la délivrance du brevet: **01.03.2017**
(21) Numéro de dépôt: 14736884.9
(22) Date de dépôt: 05.06.2014
(51) Int. Cl.: A61D 7/04, A61M 16/00

(54) **STATION D'ANESTHÉSIE POUR ANIMAUX DE LABORATOIRE ET PROCÉDÉ DE DÉTERMINATION DU TAUX DE SATURATION EN HALOGÉNÉS DES FILTRES D'UNE TELLE STATION D'ANESTHÉSIE**
ANÄSTHESIESTATION FÜR LABORTIERE UND VERFAHREN ZUR BESTIMMUNG DER HALOGENORGANISCHEN SÄTTIGUNGSRATE DER FILTER SOLCH EINER ANÄSTHESIESTATION
ANESTHESIA STATION FOR LABORATORY ANIMALS AND METHOD FOR DETERMINING THE ORGANOHALOGEN SATURATION RATE OF THE FILTERS OF SUCH AN ANESTHESIA STATION

(30) Priorité: 23.07.2013 FR 1357222
(43) Date de publication de la demande: 01.06.2016
(73) Titulaire: Anesteo, 34400 Lunel (FR)
(72) Inventeur: LE BARS, Thomas, 34400 Villetelle (FR); CARON, Sébastien, 80800 Bussy Les Daours (FR)
(74) Mandataire: IP Trust
(86) Numéro de dépôt international: PCT/FR2014/051341
(87) Numéro de publication internationale: WO 2015/011351

(56) Documents cités:
- WO-A1-2012/064245
- US-A1- 2005 263 154
- US-A1- 2012 222 556
- US-B1- 6 776 158

## Description

### DOMAINE TECHNIQUE DE L'INVENTION

L'invention concerne le domaine de l'usage d'anesthésiques gazeux destinés aux animaux de laboratoire (animaux de petites tailles) tel que des gaz anesthésiants halogénés.

L'invention concerne plus particulièrement une station d'anesthésie pour des animaux de laboratoire avec captation par filtre des halogènes contenus dans les gaz anesthésiant, et un procédé de détermination du taux de saturation en halogénés du filtre équipant une telle station d'anesthésie.

### ETAT DE LA TECHNIQUE

De manière classique en soi, une station d'anesthésie assure la distribution des gaz halogénés aux fins de l'anesthésie des animaux de laboratoire mais également l'aspiration et la filtration des gaz distribués aux fins de protéger les utilisateurs de la station.

Concernant l'opération d'anesthésie même, elle comprend de manière classique deux phases principales : une première phase d'endormissement, ou perte de conscience, appelée phase d'induction, et une phase de maintien de l'endormissement, appelée phase d'entretien. La phase d'induction consiste à délivrer à l'intérieur d'une boite, appelée boite ou chambre d'induction, les agents anesthésiques à fort débit et forte concentration afin d'endormir rapidement l'animal. La phase d'entretien consiste quant à elle à délivrer le mélange gazeux à plus faible débit et plus faible concentration aux animaux anesthésiés de manière à les maintenir endormis. La délivrance du gaz est effectuée dans ce cas à l'aide de masques.

Concernant l'aspiration et la filtration des gaz, elle est réalisée, dans les stations d'anesthésie de l'art antérieur, à l'aide de filtres retenant les halogénés, lesdits filtres étant placés dans le circuit d'aspiration en sortie de la chambre d'induction. A titre d'exemple, il peut être cité le brevet US6776158 lequel décrit un système d'anesthésie pour animaux de laboratoire comprenant un filtre à charbon actif.

Par ailleurs, afin de permettre une aspiration locale des gaz halogénés, en particulier au niveau des masques, il est d'usage d'équiper les stations d'anesthésie d'un plateau aspirant.

Les stations d'anesthésie de l'art antérieur présentent cependant l'inconvénient de nécessiter un contrôle régulier des filtres utilisés afin de vérifier qu'ils ne soient pas saturés ou n'arrivent pas à saturation en halogénés. En effet, un filtre complètement saturé en halogénés n'exerce plus sa fonction, et par conséquent expose les utilisateurs aux gaz anesthésiant en cas de fonctionnement de la station d'anesthésie.

Afin de palier cet inconvénient, certaines stations d'anesthésie de l'art antérieur prévoient des systèmes d'alertes visuels et/ou sonores. Le système d'anesthésie du brevet précédemment cité, par exemple, est équipé d'un dispositif permettant de déterminer le taux d'absorption en halogénés du filtre par mesure du taux d'halogénés en sortie du filtre, ledit dispositif étant relié à des moyens d'alarme en cas de dépassement d'un seuil de taux d'absorption prédéterminé. Un tel système d'anesthésie présente cependant l'inconvénient de ne pas permettre aux utilisateurs un contrôle à tout moment de l'état du filtre (filtre peu saturé ou proche de l'état de saturation) durant le déroulement de l'opération d'anesthésie, ces derniers n'étant avertis qu'à un niveau de seuil déterminé. Les utilisateurs n'ont en outre aucune visibilité sur l'état du filtre avant le début de l'opération d'anesthésie. L'utilisateur ne peut donc pas évaluer l'état du filtre avant le lancement de l'anesthésie et durant l'opération d'anesthésie, et de ce fait ne peut être assuré qu'il ne sera pas exposé au cours de l'anesthésie aux halogénés en cas de filtre saturé au cours de celle-ci.

L'invention vise à remédier à ces problèmes en proposant une station d'anesthésie et un procédé associé offrant un contrôle automatique du taux de saturation du filtre avant le lancement de l'anesthésie et durant celle-ci, et ce de manière fiable et sécurisée pour l'utilisateur.

### OBJET DE L'INVENTION

A cet effet, et selon un premier aspect, l'invention propose une station d'anesthésie pour un ou plusieurs animaux de laboratoire comprenant un bloc d'anesthésie relié à au moins un périphérique recevant l'animal de laboratoire, ledit bloc d'anesthésie étant apte à délivrer audit périphérique des gaz anesthésiques halogénés, des moyens d'aspiration des gaz halogénés depuis le périphérique, au moins un filtre présentant une capacité de rétention déterminée de concentration d'halogénés, ledit filtre étant agencé pour être traversé par les gaz anesthésiques halogénés en provenance du périphérique lors du fonctionnement des moyens d'aspiration, et un système de détermination automatique en temps réel du taux de saturation du filtre en halogénés, ledit système comprenant des moyens de mesure des débits instantanés des gaz anesthésiques halogénés traversant le filtre à des intervalles de temps donnés, des moyens de détermination de la concentration des gaz halogénés retenue dans le filtre en fonction des débits instantanés des gaz anesthésiques halogénés mesurés à chaque intervalle de temps donnés, et des moyens de calcul du taux de saturation du filtre à partir des concentrations de gaz anesthésiques halogénés déterminées à chaque intervalle de temps et de la capacité de rétention déterminée du filtre.

Avantageusement, les moyens de calcul sont configurés pour calculer la capacité restante de rétention de concentration d'halogénés du filtre à un instant déterminé.

Avantageusement, les moyens de calcul sont configurés pour calculer un estimatif de temps restant d'utilisation du filtre. Ce temps restant est déterminé en fonction du protocole d'utilisation identifié. Plus particulièrement, il est estimé à partir d'un historique de données d'utilisation enregistré dans une mémoire d'une unité de commande de la station d'anesthésie.

Avantageusement, la station d'anesthésie comporte des moyens d'affichage des données relatives au taux de saturation du filtre, la capacité restante de rétention de concentration d'halogénés du filtre et/ou le temps restant estimé d'utilisation du filtre.

Avantageusement, le bloc d'anesthésie est relié à au moins un équipement périphérique de distribution des gaz anesthésiques halogénés.

Avantageusement, les moyens d'aspiration et le filtre sont intégrés au bloc d'anesthésie. Il peut être également prévu que le système de détermination automatique en temps réel du taux de saturation du filtre en halogénés soit intégré au bloc d'anesthésie.

Avantageusement, la station d'anesthésie comprend un premier type de périphérique dit « d'induction » se présentant sous la forme d'une chambre hermétique. Elle peut comprendre également un deuxième type de périphérique dit « d'entretien » se présentant sous la forme d'un masque apte à recevoir un animal de laboratoire préalablement anesthésié. Il peut être prévu que la station d'anesthésie comprenne un ou plusieurs périphérique(s) additionnel(s) raccordé(s) aux moyens de ventilation. Ce périphérique peut par exemple se présenter sous la forme d'un plateau aspirant destinée à aspirer localement les gaz halogénés au niveau d'un périphérique du premier type ou du deuxième type. Il peut s'agir également d'un imageur optique tel qu'un imageur utilisant la technologie de bioluminescence en vue d'identifier et de caractériser le développement de tumeurs chez l'animal.

Avantageusement, la station d'anesthésie comprend une unité de commande 100 du fonctionnement du bloc d'anesthésie, ladite unité commandant l'arrêt de délivrance des gaz anesthésiant halogénés lorsque le taux de saturation calculé du filtre correspond au taux de saturation maximal du filtre.

Avantageusement, l'unité de commande comporte des moyens de calcul pour calculer la puissance d'aspiration délivrée par les moyens d'aspiration en fonction du volume de gaz anesthésiant délivré au périphérique.

Avantageusement, l'unité de commande comporte un lecteur RFID apte à communiquer avec une étiquette RFID portée par le filtre.

Avantageusement, le bloc d'anesthésie comporte des moyens d'alerte sonores et/ou visuels couplés à l'unité de calcul.

L'invention concerne également un procédé de détermination du taux de saturation en halogénés d'un filtre traversé par un flux de gaz anesthésiques halogénés, le filtre présentant une capacité de rétention déterminée de concentration d'halogénés, le procédé étant remarquable en ce qu'il comprend les étapes consistant à :
- mesurer le débit des gaz anesthésiques halogénés à des intervalles de temps réguliers,
- déterminer la concentration de gaz retenue dans le filtre par corrélation avec le débit mesuré à chaque intervalle de temps,
- calculer le taux de saturation du filtre par sommation des concentrations de gaz déterminé à chaque intervalle de temps et comparaisons avec la capacité de rétention déterminée du filtre.

Avantageusement, le procédé comporte une étape de détermination de la capacité restante de rétention de concentration d'halogénés à partir des données relatives au taux de saturation du filtre calculé à l'instant t et à la capacité de rétention de concentration d'halogénés.

Avantageusement, le procédé comporte une étape de détermination du temps d'utilisation restant estimé à partir des données relatives à la capacité restante de rétention de concentration d'halogénés et au débit moyen des gaz anesthésiques halogénés calculé à partir des débits instantanés des gaz halogénés mesuré à chaque intervalle de temps.

Avantageusement, le taux de saturation du filtre est calculé à partir de la sommation de la concentration de gaz déterminée à un instant donné lors de la mesure du flux instantané avec les concentrations incrémentée des gaz déterminées à chaque intervalle de temps.

### BREVE DESCRIPTION DES FIGURES

D'autres objets et avantages de l'invention apparaîtront au cours de la description qui suit, faite en référence aux dessins annexés, dans lesquels :
- la figure 1 représente un schéma de principe de la station d'anesthésie selon l'invention ;
- la figure 2 représente un synoptique de commande de la station d'anesthésie.

### DESCRIPTION DETAILLEE DES FIGURES

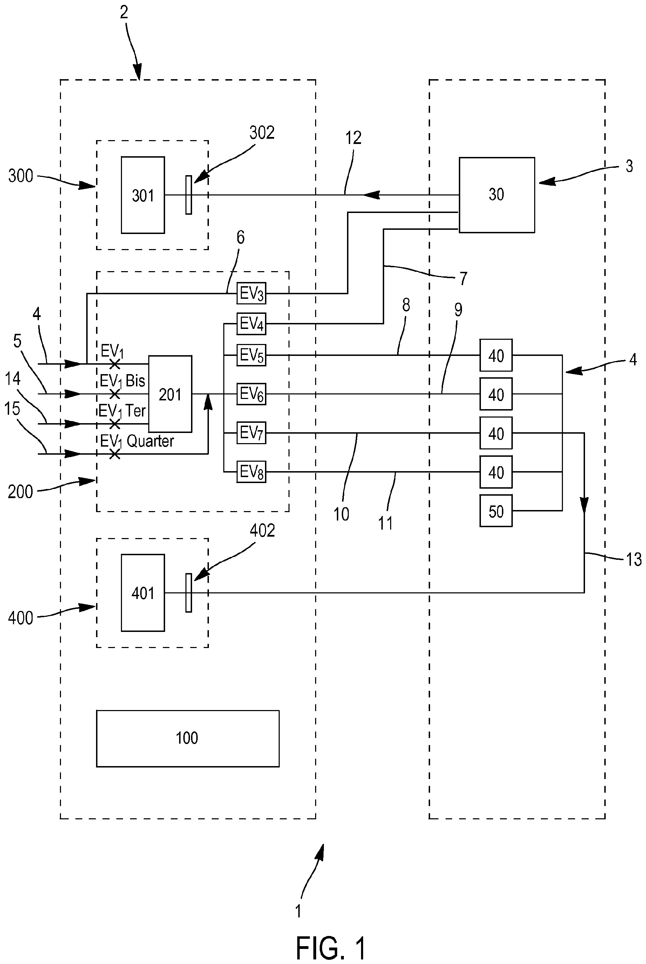

En relation avec la figure 1, il est décrit une station d'anesthésie 1 pour animaux de laboratoire tels que des rongeurs.

La station d'anesthésie 1 comprend un bloc d'anesthésie 2 relié à des périphériques 3, 4 recevant les animaux. Le bloc d'anesthésie 2 assure non seulement la distribution des gaz anesthésiant halogénés aux fins de l'anesthésie des animaux mais également l'aspiration et la filtration des gaz distribués dans les périphériques 3,4.

Dans le mode de réalisation illustré, les périphériques 3, 4 comprennent une chambre d'induction 30 pour la phase d'anesthésie dite d'induction et des masques 40 (dans l'exemple illustré, quatre masques) pour la phase d'anesthésie dite d'entretien. La chambre d'induction 30 se présente sous la forme d'une boite hermétique équipée d'une porte ou d'un couvercle soit amovible soit mobile entre une position d'ouverture pour permettre le passage des animaux dans la boite et une position de fermeture. En complément de la chambre d'induction 30 et des masques 40, la station d'anesthésie 1 comporte avantageusement un plateau aspirant 50. Un tel plateau permet d'aspirer localement les gaz halogénés au niveau d'un périphérique 3, 4, et en particulier au niveau des masques 40.

Dans l'exemple illustré, la station d'anesthésie 1 comporte six périphériques dont un plateau aspirant. Il est bien entendu évident que le nombre de ports de branchement de périphériques au bloc d'anesthésie 2 peut varier en fonction de la taille de la station souhaitée. De manière générale, la chambre d'induction 30 constitue le périphérique essentiel de la station d'anesthésie 1, les autres périphériques 40, 50 (masques 40 et plateau aspirant 50) pouvant varier d'une station à une autre selon la quantité, la taille et le type d'animaux à anesthésier.

Bien que non représenté, il peut être également prévu une station d'anesthésie comprenant plusieurs chambres d'induction.

Le bloc d'anesthésie 2 comprend un poste de distribution 200 relié à chaque périphérique 3, 4 par un circuit fluidique d'injection et deux postes d'aspiration et de filtration 300, 400, l'un étant relié par un premier circuit fluidique d'aspiration au périphérique de la phase d'induction (chambre d'induction 30), l'autre étant relié par un deuxième circuit fluidique d'aspiration aux périphériques 40, 50 de la phase d'entretien (masques 40 et plateau aspirant 50).

Dans le mode de réalisation illustré, le poste de distribution 200 du bloc d'anesthésie 2 comprend en entrée trois lignes fluidique de distribution 4, 5 et 14, chaque ligne étant destinée à transporter un gaz vers un évaporateur 201. Ainsi, le poste de distribution 200 comprend une première ligne fluidique 4 pour la distribution d'oxygène, une deuxième ligne fluidique 5 pour la distribution d'air et une troisième ligne fluidique 14 pour la distribution de protoxyde d'azote. Les gaz ainsi distribués à l'évaporateur sont des gaz porteurs qui vont permettre, seul ou mélangés les uns au autres, d'évaporer un agent anesthésiant du type Isoflurane ou Sévoflurane contenu dans l'évaporateur 201. L'agent anesthésiant, initialement introduit à l'état liquide dans l'évaporateur 201 à l'aide d'une clé de remplissage, passe ainsi à l'état gazeux sous l'action du(des) gaz porteur(s) injectés dans l'évaporateur 201. Dans le mode de réalisation illustré, la ligne fluidique 4 est pourvue d'une dérivation 6 vers la chambre d'induction pour permettre de délivrer dans la chambre d'induction 30 de l'oxygène une fois la phase d'induction achevée. Les lignes de distribution sont pourvues d'une électrovanne EV1, EV1bis et EV1ter, et la dérivation d'une électrovanne EV3.

Avantageusement, le poste de distribution 200 comporte une quatrième ligne fluidique 15 pour la distribution de dioxyde de carbone aux périphériques 3 ou 4. La ligne fluidique 15 est raccordée en sortie de l'évaporateur 201. Elle est pourvue d'une électrovanne EV1quater.

Il est bien entendu évident que le poste de distribution n'est pas limité à la configuration décrite ci-dessus, le nombre de lignes fluidique en entrée du poste de distribution pouvant varier sans sortir du cadre de l'invention. Selon une configuration a minima, il peut ainsi être prévu un poste de distribution ne distribuant qu'un seul gaz porteur, en l'espèce l'oxygène. Une seule ligne fluidique sera alors nécessaire, les autres lignes étant facultatives.

Le poste de distribution 200 comporte en sortie cinq ports permettant la connexion du bloc d'anesthésie 2 aux cinq périphériques 30, 40 délivrant les gaz anesthésiant aux animaux (chambre d'induction 30 et masques 40). La ligne d'injection 7 raccordant le poste de distribution 200 à la chambre d'induction 30 est indépendante des lignes d'injection 8 à 11, raccordant le poste de distribution 200 aux masques 40. Comme précédemment, chaque ligne de distribution est pourvue d'une électrovanne EV4 à EV8.

Comme indiqué précédemment, la station d'anesthésie 1 comprend deux postes d'aspiration/filtration 300, 400 : un premier poste 300 raccordé à la chambre d'induction 30 et un deuxième poste 400 raccordé aux masques 40 et au plateau aspirant 50. Il est bien entendu évident que le nombre de poste d'aspiration/filtration n'est pas limité à deux et qu'il peut être prévu une station d'anesthésie 1 comprenant un poste d'aspiration/filtration commun aux deux types de périphériques 30, 40 y compris le plateau aspirant 50, ou bien plus de deux postes d'aspiration. Cette dernière configuration peut notamment être prévue pour des stations d'anesthésie comprenant un nombre important de périphériques.

Chaque poste d'aspiration/filtration 300, 400 comprend un ventilateur 301, 401 raccordé en sortie d'un des périphériques 30, 40, 50 par une ligne d'aspiration. Ainsi, la chambre d'induction 30 est relié en sortie à l'un des ventilateurs 301 par une première ligne d'aspiration 12, tandis que chaque masque ainsi que le plateau aspirant 50 sont branchés à une deuxième ligne d'aspiration 13 les raccordant à l'autre ventilateur 401. Les premières et deuxième lignes d'aspiration sont indépendantes l'une de l'autre. Les ventilateurs 301, 401 sont asservis. Ils contrôlent le débit des gaz entrant dans les périphériques.

Afin de retenir la partie halogénée des gaz anesthésiant, et ainsi protéger les utilisateurs lors de l'aspiration des gaz, les circuits fluidiques d'aspiration reliant la chambre d'induction 30 et les périphériques d'entretien 40 sont pourvus respectivement d'un filtre de rétention d'halogénés 302, 402. Plus particulièrement, chaque filtre 302, 402 est agencé dans les lignes d'aspiration 12, 13, entre le périphérique concerné (chambre d'induction 30, masque et plateau aspirant 50) et le ventilateur associé 301, 401, de façon à être traversé par les gaz anesthésiques halogénés lors du fonctionnement du ventilateur associé.

Le nombre de filtres est indépendant de celui des ventilateurs. Il peut être ainsi prévu un filtre par périphérique. Selon une autre configuration, il peut être également prévu un filtre commun pour le périphérique d'induction 3 et les périphériques d'entretien 4.

Chaque filtre 302, 402 est avantageusement équipé d'une étiquette d'indentification RFID (non représentées). Chaque filtre 302, 402 présente ainsi un numéro d'identification unique lequel est associé, comme on le verra plus loin, à une interface RFID 101 ou compteur d'une unité de commande 100 intégrée dans le bloc d'anesthésie 2. Ainsi, lorsque le numéro d'identification du filtre est inconnu, un nouveau compteur est créé et initialisé par l'unité de commande 100. S'il est connu, le compteur associé est décompté périodiquement durant le fonctionnement de la pompe. L'absence de réponse de l'étiquette signifie que le filtre est absent. Un message « Filtre absent » est alors affiché sur un écran prévu à cet effet de préférence sur le bloc d'anesthésie 2. Comme on le verra plus loin, l'absence de filtre ou le décomptage total du compteur interdira tout fonctionnement du bloc d'anesthésie 2 (ie. toute distribution de gaz anesthésiant).

Avantageusement, le filtre 302, 402 est un filtre alvéolaire à flux turbulent. Le filtre 302, 402 peut être un filtre à carbone moulé et à structure alvéolaire dans lequel les gaz sont absorbés par un réactif. L'avantage d'un tel filtre est une captation optimisée des halogénés du fait de la technologie des flux turbulents.

Afin de permettre un contrôle en temps réel du filtre 302, 402 et de garantir sa fonctionnalité à l'utilisateur de la station d'anesthésie 1, cette dernière comprend un système de détermination automatique en temps réel du taux de saturation du filtre 302, 402 en halogénés. Le système de détermination automatique du taux de saturation a donc pour objectif d'évaluer le taux d'usure du filtre 302, 402 et, le cas échéant, de prévenir l'utilisateur. Pour ce faire, le système de détermination du taux de saturation comprend des moyens de mesure des débits instantanés des gaz anesthésiques halogénés traversant le filtre 302, 402 à des intervalles de temps donnés, des moyens de détermination de la concentration des gaz halogénés retenue dans le filtre 302, 402 en fonction des débits instantanés des gaz anesthésiques halogénés mesurés à chaque intervalle de temps, et des moyens de calcul du taux de saturation du filtre 302, 402 à partir des concentrations de gaz anesthésiques halogénés déterminées à chaque intervalle de temps et de la capacité de rétention déterminée du filtre 302, 402.

Avantageusement, les moyens de calcul sont configurés pour calculer la capacité restante de rétention de concentration d'halogénés du filtre 302, 402 à un instant déterminé ainsi que le temps restant estimé d'utilisation du filtre 302, 402.

Avantageusement, la station d'anesthésie 1 comporte des moyens d'affichage des données calculées, et notamment celles relatives au taux de saturation du filtre 302, 402, à la capacité restante de rétention de concentration et/ou au temps restant estimé d'utilisation du filtre 302, 402. Afin d'affiner la comptabilisation du taux d'utilisation, il peut être prévu un débitmètre implanté dans les lignes d'aspiration, en sortie du filtre 302, 402, pour mesurer le volume gazeux total filtré par le filtre 302, 402.

Avantageusement, la station d'anesthésie 1 comporte des moyens d'alerte sonores et/ou visuels couplés à l'unité de calcul.

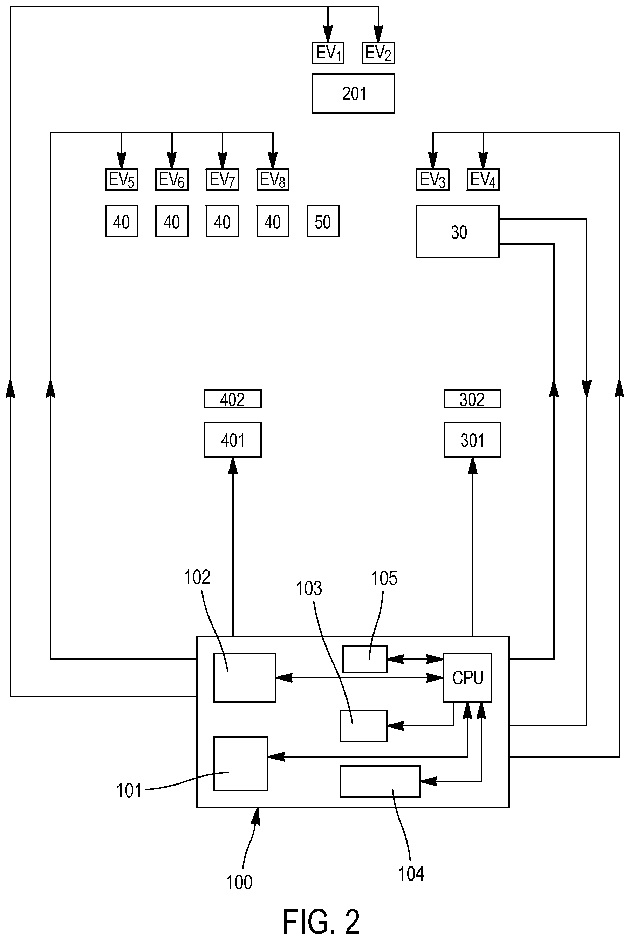

La station d'anesthésie 1 comprend en outre une unité de commande 100 intégrée au bloc d'anesthésie 2. Comme indiqué précédemment et représenté sur la figure 2, l'unité de commande 100 est configurée pour commander le fonctionnement du poste de distribution 200 (commande de l'injection de l'air ou des gaz anesthésiant dans les périphériques, commande du débit d'air et de gaz), mais également pour commander le fonctionnent des périphériques ainsi que celui des postes d'aspiration/filtration 300, 400 (commande de la puissance d'aspiration à mettre en oeuvre au niveau de la chambre d'induction 30, des masques 40 et plateau aspirant 50).

Comme illustré sur la figure 2, l'unité de commande 100 comprend une interface RFID 101 apte à communiquer avec les étiquettes RFID portées par les filtres 302 et 402, un module de contrôle de pression et de débit des gaz anesthésiant 102, un module de commande des électrovannes 103, un module d'acquisition et d'affichage 104 de données sur l'écran tactile et un bloc mémoire 105 dans lequel l'ensemble des données relatives aux précédentes séances d'anesthésie et en particulier à l'utilisation de la station (comme par exemple la durée des phases, la quantité de gaz administrée, etc.).

S'agissant de la commande des périphériques 3, 4, et en particulier la commande de la chambre d'induction 30, l'unité de commande 100 commande le verrouillage ou le déverrouillage de la porte de la chambre d'induction 30 selon que le filtre 302 associé soit détecté par l'interface RFID 301 ou non. En effet, pendant la phase d'injection, la porte (ou le couvercle) de la chambre d'induction 30 est verrouillée. L'unité de commande 100 a alors pour fonction de mettre fin à la phase d'induction (arrêt de l'injection des gaz depuis le bloc d'anesthésie 2), mais également de déclencher simultanément la phase de vidange (injection air seul et aspiration depuis la station) avant de débloquer la porte et d'autoriser l'ouverture par l'utilisateur. Cette séquence permet de garantir que la chambre d'induction 30 est totalement vidangée avant son ouverture, et donc que l'utilisateur ne sera pas exposé aux gaz anesthésiques lors de l'ouverture de la chambre d'induction 30.

L'unité de commande 100 est configurée également pour commander le verrouillage ou le déverrouillage de la porte de la chambre d'induction 30 selon le taux de saturation du filtre 301.

S'agissant de la commande du poste de distribution 200, l'unité de commande 100 commande l'arrêt de délivrance des gaz anesthésiant halogénés lorsque notamment le taux de saturation calculé du filtre 301 correspond au taux de saturation maximal de ce dernier.

Le fonctionnement de la station d'anesthésie 1 est le suivant.

Préalablement au lancement de l'anesthésie, l'utilisateur rentre, via les moyens d'acquisition ménagés sur le bloc d'anesthésie 1, les données relatives au type d'animal destiné à être anesthésié, et le cas échéant d'autres informations comme le nombre d'animaux placés dans la chambre d'induction 30, le poids de ces derniers, etc. A partir de ces informations, la station d'anesthésie détermine alors, via l'unité de commande 100, le volume optimal à injecter dans la chambre d'injection et la durée pendant laquelle l'injection devra être réalisée ainsi que le volume optimal qu'il conviendra d'aspirer une fois la phase d'induction terminée ainsi que la durée pendant laquelle l'aspiration devra être réalisée. Cette opération peut être réalisée avant ou après avoir placé les rongeurs dans la chambre d'induction 30.

Lorsque le lancement de la phase d'induction est commandé, l'unité de commande 100 va lancer, préalablement à la distribution des gaz anesthésiant selon le volume optimal préalablement déterminé, une opération de vérification de la présence du filtre 302 et de son bon fonctionnement par l'envoi d'un signal depuis l'interface RFID à l'étiquette RFID portée par le filtre 302. En cas de non réponse du filtre ou bien de la détection d'un filtre saturé en halogénés, l'unité de commande 100 interdira au poste de distribution 200 toute distribution de gaz anesthésiant. En cas de détection de la présence du filtre 302 et de l'absence de toute indication selon laquelle le filtre 302 est saturé, l'unité de commande 100 envoie des instructions au poste de distribution 200 pour la distribution de gaz anesthésiant selon le volume d'injection préalablement déterminé.

Une fois la phase d'induction achevée, il est procédé à la phase de vidange de la chambre d'induction 30 : les gaz anesthésiant sont aspirés par le poste d'aspiration associé à la chambre d'aspiration tandis que, simultanément, de l'oxygène est injecté depuis le poste de distribution 200 par la dérivation 6 dans la chambre d'induction 30. Tant que la phase de vidange n'est pas achevée, l'unité de commande 100 maintient le verrouillage de la porte (ou du couvercle). Ce n'est qu'une fois la phase de vidange terminée que l'unité de commande 100 envoie des instructions de déverrouillage de la porte (ou couvercle) de la chambre d'induction 30. Cette séquence complète permet ainsi de garantir que la chambre d'induction 30 est totalement vidangée, et que par conséquent l'utilisateur ne sera pas exposé aux gaz anesthésiques.

Les animaux sont ensuite équipés de masques 40 en vue de la phase d'entretien. Comme pour la chambre d'induction 30, l'unité de commande 100 va effectuer une opération de vérification de présence du filtre 402 associé aux deuxième poste d'aspiration/filtration 400 (filtre associé aux masques 40 et au plateau aspirant 50) et de son bon fonctionnement par l'envoi d'un signal depuis l'interface RFID à l'étiquette RFID portée par ledit filtre 402. En cas de non réponse du filtre ou bien de la détection d'un filtre saturé en halogénés, l'unité de commande 100 interdira au poste de distribution 200 toute distribution de gaz anesthésiant aux masques 40. En cas de détection de la présence du filtre 402 et de l'absence de toute indication selon laquelle le filtre 402 est saturé, l'unité de commande 100 envoie des instructions au poste de distribution 200 pour la distribution de gaz anesthésiant aux masques 40 selon le volume d'injection préalablement déterminé. Ce volume est préalablement déterminé par l'unité de commande 100 en fonction du type d'animal. En complément, l'unité de commande 100 détermine également la puissance d'aspiration à mettre en oeuvre simultanément lors de l'injection des gaz. Cette puissance est établie en fonction du volume d'injection des gaz anesthésiant dans les masques. Cela permet ainsi de garantir un captage optimum des gaz anesthésiques au niveau des masques 40, et par conséquent de garantir la non exposition des utilisateurs auxdits gaz.

Pendant toute la durée des phases d'induction et d'entretien, le taux de saturation en halogénés des filtres 302, 402 est indiqué à l'utilisateur. Pour déterminer le taux de saturation en halogénés du filtre 302, 402 traversé par un flux de gaz anesthésiques halogénés, il est procédé de la manière suivante.

On mesure tout d'abord à des intervalles de temps périodiques le débit des gaz anesthésiques halogénés traversant le filtre 302, 402. Les données relevées sont alors transmises à l'unité de commande 100 laquelle va déterminer, à l'aide d'une abaque préalablement établie et enregistrée dans une mémoire de l'unité de commande 100, la concentration de gaz retenue dans le filtre 302, 402. L'abaque définit une concentration de gaz en fonction du débit relevé. De manière générale, le débit de gaz, et donc celui du ventilateur, varie selon le périphérique utilisé. Ainsi, de manière classique en soit, le débit de gaz est plus élevé lorsque le gaz est destiné à la chambre d'induction 30 que le débit de gaz destiné aux masques. Bien entendu, le débit sera adapté en fonction du type et de la taille de l'animal à anesthésier. A chaque débit est associé un taux de concentration. Ainsi, pour un débit en phase d'induction, la concentration retenue par le filtre est de l'ordre de 4%, et pour un débit en phase d'entretien, le taux de concentration est compris entre 1 et 3% (variable selon le type et la taille de l'animal). On comprend également que lorsque le débitmètre ne relève aucun débit, la station d'anesthésie 1 est en arrêt.

Le taux de saturation du filtre 302, 402 est déterminé par sommation du taux de concentrations de gaz déterminé à chaque intervalle de temps et par comparaison avec la capacité de retenu globale du filtre 302, 402. Préférentiellement, le taux de concentration est incrémenté à chaque nouvelle mesure de débit de gaz. Le taux de saturation du filtre 302, 402 est affiché sur l'écran d'affichage du bloc d'anesthésie 2. En complément ou en remplacement, il peut être prévu d'afficher la capacité restante de rétention de concentration d'halogénés établie à partir de la capacité de rétention de concentration d'halogénés et du taux de saturation du filtre 302, 402 calculé à l'instant t.

L'unité de commande 100 procède ensuite à une estimation du temps d'utilisation restant du filtre 302, 402. Plus particulièrement, connaissant la capacité restante de rétention de concentration d'halogénés et du débit moyen des gaz anesthésiques halogénés, ce dernier étant calculé à partir des débits instantanés des gaz halogénés mesurés à chaque intervalle de temps, le temps d'utilisation est estimé à partir des données mémorisées dans le bloc mémoire 105 et relatives aux précédentes utilisations de la station

(évaluation de la récurrence d'utilisation). Ainsi, pour une même capacité restante de rétention de concentration d'halogénés du filtre, le temps d'utilisation restant pourra être jugé insuffisant ou non selon les utilisations de la station qui ont été faites précédemment.

La puissance d'aspiration à mettre en oeuvre au niveau des masques 40 est déterminée par l'unité de commande 100 en fonction du volume de gaz anesthésiant injecté. Cela permet ainsi de garantir un captage optimal des gaz anesthésiques au niveau des masques 40, et ainsi d'assurer la non exposition des utilisateurs.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Station d'anesthésie (1) pour un ou plusieurs animaux de laboratoire comprenant :
- un bloc d'anesthésie (2) relié à au moins un périphérique (3, 4) recevant l'animal de laboratoire, ledit bloc d'anesthésie (2) étant apte à délivrer audit périphérique (3, 4) des gaz anesthésiques halogénés,
- des moyens d'aspiration des gaz halogénés depuis le périphérique,
- au moins un filtre (302, 402) présentant une capacité de rétention déterminée de concentration d'halogénés, ledit filtre (302, 402) étant agencé pour être traversé par les gaz anesthésiques halogénés en provenance du périphérique (3, 4) lors du fonctionnement des moyens d'aspiration, et
- un système de détermination automatique en temps réel du taux de saturation du filtre (302, 402) en halogénés, ledit système comprenant :
o des moyens de mesure des débits instantanés des gaz anesthésiques halogénés traversant le filtre (302, 402) à des intervalles de temps donnés,
o des moyens de détermination de la concentration des gaz halogénés retenue dans le filtre (302, 402) en fonction des débits instantanés des gaz anesthésiques halogénés mesurés à chaque intervalle de temps donnés,
o des moyens de calcul du taux de saturation du filtre (302, 402) à partir des concentrations de gaz anesthésiques halogénés déterminées à chaque intervalle de temps et de la capacité de rétention déterminée du filtre (302, 402).

2. Station d'anesthésie (1) selon la revendication 1, **caractérisée en ce que** les moyens de calcul sont configurés pour calculer la capacité restante de rétention de concentration d'halogénés du filtre (302, 402) à un instant déterminé.

3. Station d'anesthésie (1) selon la revendication 1 ou la revendication 2, **caractérisée en ce que** les moyens de calcul sont configurés pour estimer le temps restant d'utilisation du filtre (302, 402) à partir d'un historique de données d'utilisation enregistré dans une mémoire d'une unité de commande de la station d'anesthésie.

4. Station d'anesthésie (1) selon l'une quelconque des revendications 1 à 3, **caractérisée en ce qu'**elle comporte des moyens d'affichage des données relatives au taux de saturation du filtre (302, 402) et à la capacité restante de rétention de concentration d'halogénés du filtre (302, 402) et/ou au temps restant estimé d'utilisation du filtre (302, 402).

5. Station d'anesthésie (1) selon l'une quelconque des revendications 1 à 4, **caractérisée en ce que** le bloc d'anesthésie (2) est relié à au moins un équipement périphérique de distribution des gaz anesthésiques halogénés.

6. Station d'anesthésie (1) selon l'une quelconque des revendications 1 à 5, **caractérisée en ce que** les moyens d'aspiration et le filtre (302, 402) sont intégrés au bloc d'anesthésie (2).

7. Station d'anesthésie (1) selon l'une quelconque des revendications 1 à 6, **caractérisée en ce qu'**elle comprend un premier type de périphérique dit « d'induction » se présentant sous la forme d'une chambre hermétique.

8. Station d'anesthésie (1) selon la revendication 7, **caractérisée en ce qu'**elle comprend un deuxième type de périphérique dit « d'entretien » se présentant sous la forme d'un masque apte à recevoir apte à recevoir un animal de laboratoire préalablement anesthésié.

9. Station d'anesthésie (1) selon la revendication 7 ou la revendication 8, **caractérisée en ce qu'**elle comprend au moins un périphérique additionnel raccordé aux moyens de ventilation se présentant sous la forme d'un plateau aspirant (50) destinée à aspirer localement les gaz halogénés au niveau d'un périphérique du premier type ou du deuxième type ou d'un imageur optique.

10. Station d'anesthésie (1) selon l'une quelconque des revendications 1 à 9, **caractérisée en ce qu'**elle comprend une unité de commande (100) du fonctionnement du bloc d'anesthésie (2), ladite unité commandant l'arrêt de délivrance des gaz anesthésiant halogénés lorsque le taux de saturation calculé du filtre (302, 402) correspond au taux de saturation maximal du filtre (302, 402).

11. Station d'anesthésie (1) selon la revendication 10, **caractérisée en ce que** l'unité de commande (100) comporte des moyens de calcul pour calculer la puissance d'aspiration délivrée par les moyens d'aspiration en fonction du volume de gaz anesthésiant délivré au périphérique.

12. Station d'anesthésie (1) selon la revendication 10 ou la revendication 11, caractérisée en ce l'unité de commande (100) comporte un lecteur RFID apte à communiquer avec une étiquette RFID portée par le filtre (302, 402).

13. Station d'anesthésie (1) selon l'une quelconque des revendications 1 à 12, **caractérisée en ce que** le bloc d'anesthésie (2) comporte des moyens d'alerte sonores et/ou visuels couplés à l'unité de calcul.

14. Procédé de détermination du taux de saturation en halogénés d'un filtre (302, 402) traversé par un flux de gaz anesthésiques halogénés utilisé pour l'anesthésie d'un ou plusieurs animaux de laboratoire, le filtre (302, 402) présentant une capacité de rétention déterminée de concentration d'halogénés, **caractérisé en ce qu'**il comprend les étapes consistant à :
- mesurer le débit des gaz anesthésiques halogénés à des intervalles de temps réguliers,
- déterminer la concentration de gaz retenue dans le filtre (302, 402) par corrélation avec le débit mesuré à chaque intervalle de temps,
- calculer le taux de saturation du filtre (302, 402) par sommation des concentrations de gaz déterminé à chaque intervalle de temps et comparaison avec la capacité de rétention déterminée du filtre (302, 402).

15. Procédé de détermination du taux de saturation en halogénés d'un filtre (302, 402) selon la revendication 14, **caractérisé en ce qu'**il comporte une étape de détermination de la capacité restante de rétention de concentration d'halogénés à partir des données relatives au taux de saturation du filtre (302, 402) calculé à l'instant t et à la capacité de rétention de concentration d'halogénés.

16. Procédé de détermination du taux de saturation en halogénés d'un filtre (302, 402) selon la revendication 15, caractérisé en qu'il comporte une étape de détermination du temps d'utilisation restant estimé à partir des données relatives à la capacité restante de rétention de concentration d'halogénés et au débit moyen des gaz anesthésiques halogénés calculé à partir des débits instantanés des gaz halogénés mesuré à chaque intervalle de temps.

17. Procédé de détermination du taux de saturation en halogénés d'un filtre (302, 402) selon l'une quelconque des revendications 14 à 16, caractérisé en que le taux de saturation du filtre (302, 402) est calculé à partir de la sommation de la concentration de gaz déterminée à un instant donné lors de la mesure du flux instantané avec les concentrations incrémentée des gaz déterminées à chaque intervalle de temps.

## Patentansprüche

1. Anästhesiestation (1) für ein oder mehrere Versuchstiere, umfassend:
- einen Anästhesiebereich (2), der an wenigstens ein Peripheriegerät (3, 4) angeschlossen ist, das das Versuchstier aufnimmt, wobei der genannte Anästhesiebereich (2) geeignet ist, halogenierte Anästhesiegase an das genannte Peripheriegerät (3, 4) auszugeben,
- Absaugmittel der halogenierten Gase ab dem Peripheriegerät,
- wenigstens einen Filter (302, 402), der eine bestimmte Rückhaltekapazität der Konzentration an halogenierten Gasen aufweist, wobei der genannte Filter (302, 402) angeordnet ist, um von den aus dem Peripheriegerät (3, 4) stammenden halogenierten Anästhesiegasen beim Betrieb der Absaugmittel durchquert zu werden und
- ein automatisches Bestimmungssystem in Echtzeit des Sättigungsniveaus des Filters (302, 402) an halogenierten Gasen, wobei das genannte System umfasst:
o Messmittel der sofortigen Durchsätze der halogenierten Anästhesiegase, die den Filter (302, 402) in bestimmten zeitlichen Intervallen durchqueren,
o Bestimmungsmittel der Konzentration der halogenierten Gase, die in dem Filter (302, 402) in Abhängigkeit von den sofortigen Durchsätzen der halogenierten Anästhesiegase zurückgehalten wird, die bei jedem bestimmten zeitlichen Intervall gemessen werden,
∘ Berechnungsmittel des Sättigungsniveaus des Filters (302, 402) ausgehend von den Konzentrationen an halogenierten Anästhesiegasen, die bei jedem zeitlichen Intervall bestimmt werden, und der bestimmten Rückhaltekapazität des Filters (302, 402).

2. Anästhesiestation (1) gemäß Anspruch 1, **dadurch gekennzeichnet, dass** die Berechnungsmittel konfiguriert sind, um die verbleibende Rückhaltekapazität der Konzentration an halogenierten Gasen des Filters (302, 402) zu einem bestimmten Zeitpunkt zu berechnen.

3. Anästhesiestation (1) gemäß Anspruch 1 oder Anspruch 2, **dadurch gekennzeichnet, dass** die Berechnungsmittel konfiguriert sind, um die verbleibende Nutzungszeit des Filters (302, 402) ausgehend von einer Historie von Nutzungsdaten zu schätzen, die in einem Speicher einer Steuereinheit der Anästhesiestation gespeichert ist.

4. Anästhesiestation (1) gemäß irgendeinem der Ansprüche 1 bis 3, **dadurch gekennzeichnet, dass** sie Anzeigemittel der Daten relativ zu dem Sättigungsniveau des Filters (302, 402) und der verbleibenden Rückhaltekapazität der Konzentration an halogenierten Gasen des Filters (302, 402) und / oder der geschätzten verbleibenden Nutzungszeit des Filters (302, 402) umfasst.

5. Anästhesiestation (1) gemäß irgendeinem der Ansprüche 1 bis 4, **dadurch gekennzeichnet, dass** der Anästhesiebereich (2) an wenigstens eine Peripherieausrüstung zur Ausgabe der halogenierten Anästhesiegase angeschlossen ist.

6. Anästhesiestation (1) gemäß irgendeinem der Ansprüche 1 bis 5, **dadurch gekennzeichnet, dass** die Absaugmittel und der Filter (302, 402) in den Anästhesiebereich (2) integriert sind.

7. Anästhesiestation (1) gemäß irgendeinem der Ansprüche 1 bis 6, **dadurch gekennzeichnet, dass** sie einen ersten Peripheriegerättyp, bezeichnet als "Induktionsperipheriegerät" umfasst, der die Form einer hermetischen Kammer darstellt.

8. Anästhesiestation (1) gemäß Anspruch 7, **dadurch gekennzeichnet, dass** sie einen zweiten Peripherietyp, bezeichnet als "Pflegeperipheriegerät" umfasst, das die Form einer Maske aufweist, die geeignet ist, ein zuvor anästhesiertes Versuchstier aufzunehmen.

9. Anästhesiestation (1) gemäß Anspruch 7 oder Anspruch 8, **dadurch gekennzeichnet, dass** sie wenigstens ein zusätzliches Peripheriegerät umfasst, das an die Belüftungsmittel angeschlossen ist, die die Form einer Absaugplatte (50) aufweisen, die dazu bestimmt ist, die halogenierten Gase an einem Peripheriegerät des ersten Typs oder des zweiten Typs oder eines optischen Bildgebers lokal abzusaugen.

10. Anästhesiestation (1) gemäß irgendeinem der Ansprüche 1 bis 9, **dadurch gekennzeichnet, dass** sie eine Steuereinheit (100) des Betriebs des Anästhesiebereichs (2) umfasst, wobei die genannte Einheit die Abschaltung der Ausgabe von halogenierten Anästhesiegasen steuert, wenn das berechnete Sättigungsniveau des Filters (302, 402) dem maximalen Sättigungsniveau des Filters (302, 402) entspricht.

11. Anästhesiestation (1) gemäß Anspruch 10, **dadurch gekennzeichnet, dass** die Steuereinheit (100) Berechnungsmittel für die Berechnung der Absaugleistung umfasst, die von den Absaugmitteln in Abhängigkeit von dem Anästhesiegasvolumen ausgegeben wird, das am Peripheriegerät ausgegeben wird.

12. Anästhesiestation (1) gemäß Anspruch 10 oder Anspruch 11, **dadurch gekennzeichnet, dass** die Steuereinheit (100) ein Lesegerät RFID umfasst, das geeignet ist, mit einem vom Filter (302, 402) getragenen Etikett zu kommunizieren.

13. Anästhesiestation (1) gemäß irgendeinem der Ansprüche 1 bis 12, **dadurch gekennzeichnet, dass** der Anästhesiebereich (2) Ton- und / oder visuelle Alarmmittel umfasst, die an die Berechnungseinheit gekoppelt sind.

14. Bestimmungsverfahren des Sättigungsniveaus an halogenierten Gasen eines Filters (302, 402), der durch einen Strom halogenierter Anästhesiegase durchquert wird, der für die Anästhesie eines oder mehrerer Versuchstiere verwendet wird, wobei der Filter (302, 402) eine bestimmte Rückhaltekapazität der Konzentration von halogenierten Gasen aufweist, **dadurch gekennzeichnet, dass** es die Stufen umfasst, bestehend aus:
- Messung des Durchsatzes der halogenierten Anästhesiegase in regelmäßigen zeitlichen Intervallen,
- Bestimmung der Rückhaltekonzentration von Gasen in dem Filter (302, 402) per Korrelation mit dem zu jedem zeitlichen Intervall gemessenen Durchsatz,
- Berechnung des Sättigungsniveaus des Filters (302, 402) per Summenbildung der zu jedem zeitlichen Intervall bestimmten Gaskonzentrationen und Vergleich mit der bestimmten Rückhaltekonzentration des Filters (302, 402).

15. Bestimmungsverfahren des Sättigungsniveaus an halogenierten Gasen eines Filters (302, 402) gemäß Anspruch 14, **dadurch gekennzeichnet, dass** es eine Bestimmungsstufe der verbleibenden Rückhaltekapazität der Konzentration an halogenierten Gasen ausgehend von den Daten relativ zum Sättigungsniveau des Filters (302, 402) umfasst, das zum Zeitpunkt t berechnet wird, und von der Rückhaltekapazität der Konzentration an halogenierten Gasen.

16. Bestimmungsverfahren des Sättigungsniveaus an halogenierten Gasen eines Filters (302, 402) gemäß Anspruch 15, **dadurch gekennzeichnet, dass** es eine Bestimmungsstufe der verbleibenden Nutzungszeit umfasst, die ausgehend von den Daten relativ zur verbleibenden Rückhaltekapazität der Konzentration an halogenierten Gasen und dem durchschnittlichen Durchsatz der halogenierten Anästhesiegase umfasst, die ausgehend von den sofortigen Durchsätzen der halogenierten Gase berechnet wird, der bei jedem zeitlichen Intervall gemessen wird.

17. Bestimmungsverfahren des Sättigungsniveaus an halogenierten Gasen eines Filters (302, 402) gemäß irgendeinem der Ansprüche 14 bis 16, **dadurch gekennzeichnet, dass** das Sättigungsniveau des Filters (302, 402) ausgehend von der Summenbildung der Gaskonzentration berechnet wird, die zu einem bestimmten Zeitpunkt bei der Messung des sofortigen Stroms mit den Konzentrationen berechnet wird, die um die bestimmten Gase zu jedem zeitlichen Intervall inkrementiert wird.

## Claims

1. An anesthesia station (1) for one or more laboratory animal(s), comprising:
- an anesthesia block (2) connected to at least one peripheral device (3, 4) receiving the laboratory animal, with said anesthesia block (2) being able to supply said peripheral device (3, 4) with halogenated anesthetic gases,
- means for aspirating the halogenated gases from the peripheral device,
- at least one filter (302, 402) having a determined organohalogen concentration retention capacity, with said filter (302, 402) being so arranged as to be crossed by the halogenated anesthetic gases from the peripheral device (3, 4) during the operation of the aspiration means, and
- a system for the automatic determination, in real-time, of the organohalogen saturation rate of the filter (302, 402), with said system comprising:
o means for measuring the instantaneous flow rates of the halogenated anesthetic gases going through the filter (302, 402) at given time intervals,
o means for determining the retained halogenated gases concentration in the filter (302, 402) according to the instantaneous flow rates of the halogenated anesthetic gases measured at each given time interval,
o means for calculating the saturation rate of the filter (302, 402) from the halogenated anesthetic gases concentrations determined at each time interval and the determined storage capacity of the filter (302, 402).

2. An anesthesia station (1) according to claim 1, **characterized in that** the calculation means are so configured as to calculate the remaining organohalogen concentration retention capacity of the filter (302, 402) at a determined time.

3. An anesthesia station (1) according to claim 1 or claim 2, **characterized in that** the calculation means are so configured as to estimate the time remaining for using the filter (302, 402) from a history of utilization data saved in a memory of a control unit of the anesthesia station.

4. An anesthesia station (1) according to any one of claims 1 to 3, **characterized in that** it comprises means for displaying data relating to the saturation rate of the filter (302, 402) and the remaining organohalogen concentration retention capacity of the filter (302, 402) and/or the estimated time remaining for using the filter (302, 402).

5. An anesthesia station (1) according to any one of claims 1 to 4, **characterized in that** the anesthesia block (2) is connected to at least one peripheral device distributing halogenated anesthetic gases.

6. An anesthesia station (1) according to any one of claims 1 to 5, **characterized in that** the aspiration means and the filter (302, 402) are integrated in the anesthesia block (2).

7. An anesthesia station (1) according to any one of claims 1 to 6, **characterized in that** it comprises a first so-called "induction" type of device in the form of a sealed chamber.

8. An anesthesia station (1) according to claim 7, **characterized in that** it comprises a second so-called "maintenance" type of device in the form of a mask suitable for receiving a previously anesthetized laboratory animal.

9. An anesthesia station (1) according to claim 7 or claim 8, **characterized in that** it comprises at least one additional peripheral device connected to the ventilation means in the form of a suction plate (50) intended to locally suck the halogenated gases at a peripheral device of the first type or of the second type or of an optical imager.

10. An anesthesia station (1) according to any one of claims 1 to 9, **characterized in that** it comprises a unit (100) controlling the operation of the anesthesia block (2), with said unit controlling the stopping of the delivery of the halogenated anesthetic gases when the calculated saturation rate of the filter (302, 402) is the maximum saturation rate of the filter (302, 402).

11. An anesthesia station (1) according to claim 10, **characterized in that** the control unit (100) comprises calculation means for calculating the suction power delivered by the suction means according to the volume of anesthetic gas delivered to the peripheral device.

12. An anesthesia station (1) according to claim 10 or claim 11, **characterized in that** the control unit (100) comprises an RFID reader adapted to communicate with an RFID tag carried by the filter (302, 402).

13. An anesthesia station (1) according to any one of claims 1 to 12, **characterized in that** the anesthesia block (2) is provided with sound and/or visual warning means coupled to the calculation unit.

14. A method for determining the organohalogen saturation rate of the filter (302, 402) crossed by a flow of halogenated anesthetic gases used for the anesthesia of one or more laboratory animal(s), with the filter (302, 402) having a specific organohalogen concentration retention capacity, **characterized in that** it comprises the steps of:
- measuring the flow of the halogenated anesthetic gases at regular time intervals,
- determining the retained gas concentration in the filter (302, 402) by correlation with the flow rate measured at each time interval,
- calculating the saturation rate of the filter (302, 402) by summing the gas concentrations determined at each time interval and comparing with the predetermined retention capacity of the filter (302, 402).

15. A method for determining the organohalogen saturation rate of a filter (302, 402) according to claim 14, **characterized in that** it comprises a step of determining the remaining organohalogen concentration retention capacity from the data relating to the saturation rate of the filter (302, 402) calculated at a time t and the organohalogen concentration retention capacity.

16. A method for determining an organohalogen saturation rate of a filter (302, 402) according to claim 15, **characterized in that** it comprises a step of determining the estimated remaining utilization time from the data relating to the remaining organohalogen concentration retention capacity and to the average flow rate of the halogenated anesthetic gases calculated from the instantaneous flow rates of the halogenated gases measured at each time interval.

17. A method for determining an organohalogen saturation rate of the filter (302, 402) according to any one of claims 14 to 16, **characterized in that** the saturation rate of the filter (302, 402) is calculated from the summing of the gas concentration determined at a given time during the measuring of the instantaneous flow with the determined gas concentrations incremented at each time interval.
